**Europäisches Patentamt**

**European Patent Office**

**Office européen des brevets**

(19)

(11) Numéro de publication: **0 036 345 B1**

(12) **FASCICULE DE BREVET EUROPEEN**

(45) Date de publication du fascicule du brevet:
**12.10.83**

(51) Int. Cl.³: **A 61 K 9/50**

(21) Numéro de dépôt: **81400274.7**

(22) Date de dépôt: **20.02.81**

(54) **Nouveau procédé d'enrobage d'au moins un principe actif médicamenteux, les nouveaux médicaments ainsi obtenus, et les compositions les renfermant.**

(30) Priorité: **26.02.80 FR 8004199**

(43) Date de publication de la demande:
**23.09.81 Bulletin 81/38**

(45) Mention de la délivrance du brevet:
**12.10.83 Bulletin 83/41**

(84) Etats contractants désignés:
**AT BE CH DE GB IT LI NL SE**

(56) Documents cités:
**FR-A-2 084 198**
**LU-A-72 439**
**US-A-3 922 379**
**«Chemical Abstracts», vol. 90, N° 2, 8 janvier 1979, p. 37, abrégé 7232u Columbus, Ohio, US.**
**«Remington's Pharmaceutical Sciences», 15ᵉ éd., 1975. Mack Publishing Co., Easton, PA, USA, pp. 314, 315, 1611.**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Ser, Jacques, 19, avenue Detouche, F-93250 Villemomble (FR)**
Inventeur: **Rineau, Jean-Claude, 6, rue des Bergeries, F-93130 Noisy le Sec (FR)**

(74) Mandataire: **Vieillefosse, Jean-Claude et al, ROUSSEL-UCLAF Boîte postale no. 9 102, route de Noisy, F-93230 Romainville (FR)**

**Nouveau procédé d'enrobage d'au moins un principe actif médicamenteux, les nouveaux médicaments ainsi obtenus, et les compositions les renfermant**

La présente invention concerne un nouveau procédé d'enrobage d'au moins un principe actif médicamenteux, les nouveaux médicaments ainsi obtenus et les compositions les renfermant.

L'administration par voie orale de médicaments, notamment aux enfants, pose des problèmes. Ces mêmes problèmes peuvent d'ailleurs se poser chez bien des adultes. En effet, adultes et enfants, souvent, ne peuvent pas avaler — ou refusent d'avaler — un médicament présenté sous forme de gélule ou de comprimé, même de petite taille; il est plus facile de leur faire accepter un médicament présenté sous une forme liquide, telle que la forme sirop ou suspension, ou obtenu en dispersant dans un liquide le contenu d'une gélule ou un comprimé écrasé.

Cependant, certains principes actifs ont un goût si désagréable lorsqu'ils sont présentés sous ces formes que le sirop de sucre, les édulcorants ou les aromatisants ne suffisent pas à les rendre acceptables par le malade.

C'est ainsi que la titulaire a étudié un grand nombre de procédés d'enrobage de principes actifs pulvérisés.

On avait déjà décrit, dans la littérature, des procédés d'enrobage d'érythromycine ou de ses dérivés. La demande de brevet luxembourgeois No 72439 (C.E.R.T.A.) décrit, par exemple, un procédé d'enrobage d'érythromycine exigeant au préalable la dissolution de celle-ci; le brevet des Etats-Unis d'Amérique No 3922379 (Abbott) décrit la préparation de microcapsules de dérivés de l'érythromycine par utilisation d'une solution d'albumine.

Le brevet français No 2084198 (Bayer) décrit la préparation de microcapsules de solides ou de liquides à l'aide d'une solution aqueuse d'un polymère hydrophile. Le «Remington's Pharmaceutical Sciences», 15e éd., 1975, Mack Publishing Company, Easton, Penn., USA, décrit l'enrobage d'agents hydrophobes à l'aide d'un colloïde hydrophile.

La titulaire a essayé des enrobages en turbine et atomisation, à base d'hydroxypropylméthylcellulose, de gélifiants, de pectines, de sirops de sucre; elle a expérimenté la technique de microencapsulation à l'aide de Tylose® C 30, de carboxyméthylcellulose sodique, de gélatine et de gomme arabique, ainsi que la technique de fixation sur résine échangeuse d'ions (par exemple celle connue sous le nom d'IRP 64). Tous ces essais furent des échecs, totaux ou partiels.

Certaines matières plastiques, telles les résines acryliques de type Eudragit®, sont connues pour leur aptitude à former des films d'enrobage; quelques-unes de ces résines existent en suspension aqueuse, et le film d'enrobage se forme par évaporation de l'eau de la suspension. La titulaire a essayé d'utiliser de telles suspensions aqueuses, comme celles commercialisées sous les noms Eudragit® E 30 D et L 30 D par la société Röhm Pharma (Allemagne fédérale), pour réaliser un enrobage par atomisation; cette technique n'a pas été utilisable, car la suspension de résine et du principe actif s'est prise en masse dans le réservoir et a bouché le disperseur.

C'est en poursuivant ces derniers travaux que la titulaire a découvert un nouveau procédé d'enrobage d'au moins un principe actif médicamenteux, dans lequel l'enrobage se forme non par évaporation de l'eau d'une suspension aqueuse d'un agent hydrophobe solide, mais par floculation, au sein de la suspension. En fonction d'une épaisseur croissante du film, le goût du ou des principes actifs est d'abord efficacement masqué; ensuite, un effet de libération prolongée du ou des principes actifs est en outre obtenu.

La présente invention a ainsi pour objet un nouveau procédé d'enrobage d'au moins un principe actif médicamenteux solide, peu hydrosoluble et se présentant sous forme finement divisée, caractérisé en ce que l'on mélange sous agitation ledit principe actif et une dispersion aqueuse d'un ou plusieurs agents hydrophobes solides pharmaceutiquement acceptables, fait floculer ledit agent hydrophobe à l'aide d'au moins un moyen physique ou chimique ou une combinaison de ces moyens, sépare le principe actif ainsi enrobé et sèche.

Le procédé peut s'appliquer à un principe actif isolé ou à un mélange compatible de principes actifs. Le ou les principes actifs doivent répondre aux critères ci-dessus indiqués, et développés ci-après.

On entend par principe actif médicamenteux solide peu hydrosoluble un principe actif médicamenteux dont une partie en poids est soluble dans plus de 100 parties d'eau en volume, à 20°C.

Par principe actif médicamenteux se présentant sous forme finement divisée, on entend un principe actif dont la taille des particules peut être comprise, par exemple, entre 1 et 500 $\mu$; les particules peuvent, par exemple, avoir une forme d'aiguilles ou une forme tabulaire. Dans le cas d'une association de principes actifs, il est préférable que leur granulométrie soit du même ordre de grandeur.

Les agents hydrophobes solides selon l'invention, en dispersion aqueuse, sont susceptibles de floculer dans certaines conditions physiques ou chimiques, telles que la chaleur, le froid, l'action des électrolytes ou les changements de pH, et doivent être pharmaceutiquement acceptables.

L'agent hydrophobe solide peut être, par exemple, un acide gras, un alcool gras, ou un ester gras, utilisé seul ou en mélanges. La molécule d'acide gras peut contenir de 10 à 22 atomes de carbone, et cet acide est, par exemple, l'acide stéarique, palmitique ou myristique.

Les molécules des alcools gras renferment de 14 à 30 atomes de carbone et ces alcools sont par exemple l'alcool cétylique, stéarylique ou myricylique.

Les esters peuvent être des mono-, di- ou triesters de glycéryle tels que le mono-, di- ou tristéarate de glycéryle, le mono-, di- ou tripalmitate de glycéryle ou le mono-, di- ou trilaurate de glycéryle.

L'agent hydrophobe solide selon l'invention peut être également un éther de cellulose et notamment l'éthylcellulose. Une suspension d'éthylcellulose dans l'eau a spécialement été conçue pour l'industrie pharmaceutique, il s'agit de la suspension commercialisée sous le nom Aquacoat® par la société FMC Corporation.

L'agent hydrophobe solide selon l'invention peut être également une substance cireuse telle qu'un ester d'un acide gras dont la molécule renferme de 12 à 30 atomes de carbone, et d'un alcool gras dont la molécule renferme de 12 à 30 atomes de carbone, ou un mélange de ces esters. On peut citer comme exemple le palmitate de myricyle, la cire de spermaceti, la cire de carnauba, le palmitate de stéaryle ou le laurate de lauryle.

L'agent hydrophobe solide selon l'invention est de préférence une résine acrylique.

Les dispersions aqueuses de matières plastiques sont bien connues, ainsi que leurs procédés de préparation, notamment dans l'industrie des peintures. Il s'agit de suspensions stables très fines, de particules sous-microscopiques, la plupart sphériques, de diamètre compris entre $10^{-2}$ et $1\mu$, obtenues par le processus d'émulsion par polymérisation. Certaines de ces suspensions sont spécialement conçues pour l'industrie pharmaceutique; il s'agit par exemple des suspensions aqueuses d'un polymérisat d'esters des acides méthacrylique et acrylique commercialisées sous le nom Eudragit® E 30 D, ou d'un polymérisat anionique à base d'acide méthacrylique commercialisée sous le nom Eudragit® L 30 D par la société Röhm Pharma (Darmstadt, Allemagne fédérale).

Lors de la floculation, les grains de résine se rassemblent autour des particules solides du ou des principes actifs maintenus en suspension par agitation, et les enrobent ainsi.

Une dispersion aqueuse d'un agent hydrophobe solide est sensible à certains facteurs, tels les électrolytes, les changements de pH, la chaleur ou le froid; ces facteurs peuvent provoquer la floculation dudit agent en suspension par soudure des grains entre eux.

On peut séparer des eaux mères le principe actif ainsi enrobé, par exemple par décantation ou centrifugation, mais de préférence par filtration. La filtration est avantageusement réalisée à l'aide d'un Buchner et peut être accélérée à l'aide du vide ou de la pression. Le solide retenu dans le filtre peut être séché tel quel, ou, si désiré, peut être préalablement lavé, par exemple, à l'aide d'eau purifiée.

Le séchage peut être réalisé selon les procédés classiques, par exemple par séchage en étuve ventilée, avec ou sans un chauffage approprié au principe actif et à l'agent hydrophobe solide.

Le principe actif enrobé sec peut alors être calibré, par exemple à l'aide d'une granuleuse, et utilisé comme un principe actif habituel non enrobé, pour une mise en forme pharmaceutique classique pour la voie orale, telle que la forme comprimé, gélule, sirop ou suspension, en évitant cependant l'emploi des solvants de l'agent hydrophobe.

Dans des conditions de réalisation préférentielles du procédé ci-dessus décrit, le moyen physique de floculation utilisé est la variation de température, et le moyen chimique de floculation utilisé est un électrolyte, de préférence le chlorure de sodium. On combine très avantageusement l'utilisation de la chaleur et d'un électrolyte.

Certains principes actifs sont difficilement mouillables, il est dans ce cas particulièrement avantageux d'ajouter un agent mouillant au mélange de l'agent hydrophobe solide et du principe actif. On utilise par exemple un agent surfactif à raison, par exemple, de 0,2 à 1%. On obtient ainsi une meilleure suspension du principe actif dans le mélange. On retient tout particulièrement le produit commercialisé sous le nom Pluronic® F 68 par Ugine-Kuhlmann.

Pour obtenir un film d'enrobage fin et solide, enrobant bien les angles des particules du ou des principes actifs, il est vivement recommandé d'incorporer au mélange un agent plastifiant, comme par exemple un polyoxyéthylèneglycol 4000 ou 6000, ce dernier de préférence. Il est également possible d'utiliser d'autres plastifiants hydrosolubles ou dispersibles tels que la triacétine; on préfère néanmoins les plastifiants hydrosolubles, à raison d'environ 0,2 à 1% en poids du mélange.

Il est aussi préférable d'ajouter au mélange un agent antimousse, par exemple la silicone antimousse connue sous le nom de Silicone Antimousse 30, obl N, par exemple à raison de 0,01%. Il est cependant possible d'utiliser par exemple la Silicone ASE2 (Wacker, Allemagne fédérale).

La quantité d'agent hydrophobe solide à mettre en œuvre rapportée au poids des particules du principe actif à recouvrir est extrêmement variable; à masse égale, un produit finement pulvérisé aura une surface spécifique beaucoup plus importante que le même produit en un seul bloc, et nécessitera en conséquence une beaucoup plus importante quantité dudit agent pour obtenir la même épaisseur d'enrobage, donc le même effet. La détermination précise de la quantité d'agent hydrophobe solide à mettre en œuvre est du domaine de l'homme de l'art. Pour des cristaux en forme d'aiguilles d'un diamètre de 3 μ et d'une longueur moyenne de 15 μ, l'agent hydrophobe solide d'enrobage peut représenter par exemple de 1 à 60% du poids de la particule enrobée; les faibles proportions, par exemple de 1 à 5%, correspondent à des particules dont le goût est efficacement masqué, et un effet de libération prolongée s'ajoute en augmentant la proportion d'agent hydrophobe solide. Il va sans dire que, dans le cas de particules beaucoup plus grosses, par exemple visibles à l'œil nu, la proportion de l'agent hydrophobe solide rapportée au poids de la particule enrobée est beaucoup plus faible.

Selon un autre aspect de l'invention, l'on incorpore en outre au mélange un additif hydrosoluble ou hydrophile. On obtient alors un médicament au goût masqué, dont le principe actif est rapidement libéré au niveau stomacal. Cette libération rapide est particulièrement recherchée dans le cas de substances dont l'absorption est effectuée dans la partie haute du tube digestif, par exemple pour des antibiotiques tels que la propionylérythromycine ou la doxycycline.

Dans des conditions de réalisation préférées du procédé ci-dessus décrit, le mélange renferme environ 1 à 60 parties d'un principe actif, qui est la propionylérythromycine, pour une partie de résine acrylique. La propionylérythromycine a été décrite par Stephens et Conine («Antibiot. Ann.», 1958-59, 346).

Dans une mise en œuvre particulière de ce dernier procédé, le mélange renferme environ 5 à 15 parties de propionylérythromycine, et 5 à 15 parties d'un additif hydrosoluble ou hydrophile, qui est le bicarbonate de sodium, pour une partie de la résine acrylique neutre constituant, en suspension aqueuse, l'Eudragit® E 30 D.

La présente invention a également pour objet de nouveaux médicaments, caractérisés en ce qu'ils sont constitués d'au moins un principe actif médicamenteux solide, peu hydrosoluble, et sous forme finement divisée, ledit principe actif étant recouvert d'un enrobage constitué du floculat d'une dispersion aqueuse d'un ou plusieurs agents hydrophobes solides pharmaceutiquement acceptables.

Parmi les médicaments objets de l'invention, on retient particulièrement ceux caractérisés en ce que l'agent hydrophobe solide est la résine acrylique neutre constituant, en suspension aqueuse, l'Eudragit® E 30 D.

Dans de tels médicaments, le goût du principe actif est masqué, et la libération du principe actif peut être étalée sur plusieurs heures si désiré.

Parmi les médicaments objets de l'invention, on retient également ceux caractérisés en ce que l'enrobage renferme en outre au moins un additif hydrosoluble ou hydrophile. Les additifs hydrosolubles ou hydrophiles peuvent être, par exemple, des produits tels que le lactose, le sucre, l'amidon, la polyvinylpyrrolidone, le chlorure de sodium ou le bicarbonate de sodium. Ils peuvent être ajoutés dans une proportion allant jusqu'à environ 20 fois celle de l'agent hydrophobe solide exprimé en matière sèche.

Dans ces derniers médicaments, le goût du principe actif est masqué, mais le principe actif est libéré dans l'estomac, d'autant plus rapidement que la proportion d'additifs est importante.

Parmi ces derniers médicaments, on retient de préférence ceux caractérisés en ce que l'additif hydrosoluble ou hydrophile est choisi dans le groupe constitué par le bicarbonate de sodium et le chlorure de sodium.

Des médicaments particulièrement préférés selon l'invention sont ceux caractérisés en ce que l'enrobage renferme, en outre, un agent plastifiant. En effet, les particules du principe actif à enrober peuvent avoir une forme très irrégulière, et l'addition d'un plastifiant permet d'avoir un film d'enrobage à la fois fin et solide, épousant bien les angles des particules de principe actif. On peut utiliser, par exemple, la triacétine ou les polyoxyéthylèneglycols 4000 ou 6000. Les plastifiants hydrosolubles à raison d'environ 0,2 à 1% du mélange, en poids, comme le polyoxyéthylèneglycol 6000, sont particulièrement préférés.

Des médicaments tout particulièrement préférés, selon l'invention, sont ceux pour lesquels le principe actif est la propionylérythromycine, dont les cristaux, de forme tabulaire, ont une taille comprise entre quelques microns et une centaine de microns. Toutefois, par chauffage en milieu aqueux, ces cristaux prennent la forme d'aiguilles d'un diamètre d'environ 3 μ et longues de quelques microns à une centaine de microns.

Parmi ces derniers médicaments, on retient de préférence ceux caractérisés en ce que l'agent hydrophobe solide est la résine acrylique neutre constituant, en suspension aqueuse, l'Eudragit® E 30 D et représente, en poids sec, de 2 à 15% du poids de la propionylérythromycine.

Les médicaments selon l'invention sont, de plus, utilisables dans d'autres domaines que ceux précédemment cités, étant donné que les nouveaux médicaments enrobés obtenus peuvent être utilisés comme le même principe actif non enrobé qu'ils renferment.

La présente invention a enfin pour objet les nouvelles compositions pharmaceutiques destinées à la voie orale, caractérisées en ce qu'elles renferment l'un au moins des médicaments tels que définis ci-dessus.

Ces compositions pharmaceutiques peuvent être, par exemple, solides ou liquides, et se présenter sous les formes pharmaceutiques pour la voie orale couramment utilisées en médecine humaine, comme par exemple les comprimés simples ou dragéifiés, les gélules, les granulés. Elles sont préparées selon les méthodes usuelles. Le ou les principes actifs enrobés peuvent y être incorporés à des excipients habituellement employés dans ces compositions pharmaceutiques, tels que le talc, la gomme arabique, le lactose, l'amidon, le stéarate de magnésium, les véhicules aqueux, les divers agents mouillants, dispersants ou émulsifiants, les conservateurs.

Les exemples suivants illustrent l'invention sans toutefois la limiter.

*Exemple 1: Préparation de propionylérythromycine enrobée*

On introduit dans un cuiseur à sirop à double enveloppe, sous forte agitation (700 tr/min), 108 l d'eau purifiée, 260 g de Pluronic® F 68 et 390 g de polyoxyéthylèneglycol 6000. On agite pendant 10 min jusqu'à dissolution complète, ajoute alors 13 kg de bicarbonate de sodium, agite pendant 30 min, ajoute 3,250 kg de dispersion aqueuse d'Eudragit® E 30 D (correspondant à 975 g de vernis sec), ajoute 13 kg de propionylérythromycine à 890 UI/mg, puis ajoute, après 10 min, toujours sous agitation, une suspension de 8,6 g de silicone antimousse 30, obl N, dans 174 ml d'eau purifiée. On laisse 45 min sous agitation, chauffe la préparation à l'aide de vapeur d'eau dans le double fond, de manière à obtenir une température de 55°C en 1 h, en continuant l'agitation. On maintient cette température pendant 15 min, ajoute alors une solution de 1,3 kg de chlorure de sodium dans 6,5 l d'eau purifiée non chauffée, agite encore pendant 45 min à 55°C, refroidit le mélange à 15-20°C par circulation d'eau à 10°C pendant 35 min et essore sous pression réduite sur des Buchners en inox équipés d'une toile en polypropylène. On empâte le gâteau d'essorage pendant 10 min sous agitation mécanique, à l'aide de 44 l d'eau purifiée, filtre à nouveau sur Buchner, rince avec 21 l d'eau, essore et sèche en étuve ventilée à 50°C. Après 20 h de séchage, on passe le produit sur une granuleuse équipée d'une grille de 3 mm d'ouverture de maille, sèche à nouveau en étuve ventilée pendant 3 h, passe alors sur une granuleuse équipée d'une grille d'ouverture de mailles de 400 µ, et obtient ainsi 13,870 kg de propionylérythromycine traitée, titrant 807 UI/mg dont le goût est masqué.

*Exemple 2: Préparation de propionylérythromycine enrobée*

On introduit dans un becher 842 ml d'eau purifiée, puis, sous agitation, 6 g de polyoxyéthylèneglycol 6000 et 2 g de Pluronic® F 68, agite pendant 5 min, ajoute alors 50 g d'Eudragit® L 30 D, agite pendant 10 min, ajoute ensuite 100 g de propionylérythromycine, agite pendant 15 min, puis chauffe à 55°C en 20 min et maintient 15 min cette température. On ajoute alors 10 g de chlorure de sodium, chauffe encore à 55°C pendant 45 min, refroidit ensuite à 20°C, filtre sur un Buchner avec toile de coton, rince le gâteau de filtration avec 500 ml d'eau, sèche pendant 16 h à 50°C en étuve ventilée, passe sur un tamis à ouverture de maille de 400 µ, et obtient 108,9 g de propionylérythromycine traitée dont le goût est masqué, titrant 732 UI/mg.

*Exemple 3: Préparation de comprimés de propionylérythromycine enrobée*

On mélange 68,3 g de propionylérythromycine traitée obtenus à l'exemple 2, 21,4 g d'Avicel® pH 101, 9,1 g d'amidon de maïs et 1,6 g de saccharinate de sodium, introduit alors 60 g d'empois et 1,54 g d'amidon de maïs à 50°C, rince avec 50 ml d'eau, granule sur un tamis de 1,6 mm d'ouverture de maille, sèche pendant 6 h en étuve ventilée à 50°C, passe sur un granulateur de 1 mm d'ouverture de maille, et obtient 100 g de granulés. On mélange les granulés obtenus avec 7 g de polvaromas ananas orange 10 A 77, 27,7 g d'Avicel® pH 101, 8,3 g d'amidon, 1,6 g d'Aérosil® 200 pendant 30 min, ajoute 1,2 g de talc, et 2 g de stéarate de magnésium, mélange encore pendant 5 min et passe alors sur une machine à comprimer.

On obtient des comprimés d'une dureté comprise entre 6,6 et 7,2 kg, se délitant en 20 à 25 s dans de l'eau à 23°C; le goût de la suspension est assez bon, peu sucré.

*Exemple 4: Préparation de comprimés de propionylérythromycine enrobée*

En utilisant un procédé identique à celui décrit dans l'exemple 3, on a préparé des comprimés répondant à la formule:

| | |
|---|---|
| Propionylérythromycine traitée obtenue à l'exemple 1 | 310 mg |
| Aérosil® 200 | 8 mg |
| Amidon de maïs | 90 mg |
| Polvaromas ananas orange 10 A 77 | 35 mg |
| Saccharinate de sodium | 8 mg |
| Stéarate de magnésium | 6 mg |
| Talc | 6 mg |
| Avicel® pH 101 q.s.p. un comprimé terminé à | 740 mg |

pour une dureté de 6 à 8,6 kg, en comprimé se délitant en 35 et 46 s environ.

*Exemple 5: Préparation de propionylérythromycine enrobée*

On dissout 0,75 g de polyoxyéthylèneglycol 6000 et 1 g de Pluronic® F 68 dans 600 ml d'eau, introduit 6,25 g d'Eudragit® E 30 D, ajoute 100 g de propionylérythromycine, agite pendant 15 min, amène la température à 55°C sous agitation, maintient cette température pendant 1 h, avec introduction, au bout de 5 min, d'une solution de 10 g de chlorure de sodium dans 50 ml d'eau, refroidit à 20-22°C, maintient 1 h sous agitation, filtre sur Buchner, lave avec 500 ml d'eau et sèche pendant 12 h en étuve ventilée à 50°C. On passe le produit séché sur tamis à ouverture de maille de 400 µ et obtient 99 g de propionylérythromycine enrobée dont la majeure partie de l'amertume est masquée.

*Exemple 6: Préparation de propionylérythromycine enrobée*

On mélange 7,2 l d'eau, 240 g de polyoxyéthylène gylcol 6000, 20 g de Pluronic® F 68, agite pendant 5 min, introduit 2 kg d'Eudragit® E 30 D et rince avec 0,8 l d'eau. On agite pendant 5 min, introduit 1 kg de propionylérythromycine, agite pendant 30 min, chauffe à 75°C en 80 min, maintient cette température pendant 15 min, toujours sous agitation, refroidit à température ambiante, agite encore pendant 1 h, filtre sous pression réduite sur Buchner, empâte sous agitation avec 4,5 l d'eau pendant 30 min, essore, rince avec 1 l d'eau, laisse essorer pendant 1 h, granule, sèche pendant 15 h à 50°C en étuve ventilée, passe sur tamis de 800 µ, puis de 400 µ d'ouverture de maille et obtient 1,513 kg du produit attendu.

*Exemple 7: Préparation de comprimés de propionylérythromycine enrobée*

On a préparé par compression directe des comprimés de 1 g répondant à la formule:

| | |
|---|---|
| Propionylérythromycine de l'exemple 6 | 457 mg |
| Avicel® pH 101 | 196,5 mg |

| Aérosil® 200 | 15 mg |
| Amidon de maïs | 90 mg |
| Farine de caroube | 222 mg |
| Menthol | 0,5 mg |
| Cacao soluble | 10 mg |
| Saccharinate de sodium | 3 mg |
| Talc | 3 mg |
| Stéarate de magnésium | 3 mg |

Les comprimés obtenus ont une dureté comprise entre 16 et 20 kg, et se délitent dans l'eau en 45 à 75 s à température ambiante.

**Revendications** pour les Etats contractants: BE, CH, DE, GB, IT, LI, NL, SE

1. Procédé d'enrobage d'au moins un principe actif médicamenteux solide, peu hydrosoluble et se présentant sous forme finement divisée, caractérisé en ce que l'on mélange sous agitation ledit principe actif et une dispersion aqueuse d'un ou plusieurs agents hydrophobes solides pharmaceutiquement acceptables, fait floculer ledit agent hydrophobe à l'aide d'au moins un moyen physique ou chimique ou une combinaison de ces moyens, sépare le principe actif ainsi enrobé et sèche.

2. Procédé selon la revendication 1, caractérisé en ce que l'agent hydrophobe solide est une résine acrylique.

3. Procédé selon l'une des revendications 1 ou 2, caractérisé en ce que le moyen physique de floculation utilisé est la variation de température.

4. Procédé selon l'une des revendications 1, 2 ou 3, caractérisé en ce que le moyen chimique de floculation utilisé est un électrolyte.

5. Procédé selon la revendication 4, caractérisé en ce que l'électrolyte est le chlorure de sodium.

6. Procédé selon l'une des revendications 1 ou 2, caractérisé en ce que, lors de l'opération de mélange, l'on ajoute en outre un agent mouillant.

7. Procédé selon l'une des revendications 1 ou 2, caractérisé en ce que, lors de l'opératioan de mélange, l'on ajoute en outre un agent plastifiant.

8. Procédé selon l'une des revendications 1 ou 2, caractérisé en ce que, lors de l'opération de mélange, l'on ajoute en outre un agent antimousse.

9. Procédé selon l'une des revendications 1 ou 2, caractérisé en ce que, lors de l'opération de mélange, l'on incorpore en outre au moins un additif hydrosoluble ou hydrophile.

10. Procédé selon la revendication 2, caractérisé en ce que le mélange renferme environ 1 à 60 parties d'un principe actif qui est la propionylérythromycine pour une partie de résine acrylique.

11. Procédé selon l'une des revendications 9 ou 10, caractérisé en ce que le mélange renferme environ 5 à 15 parties d'un principe actif qui est la propionylérythromycine et 5 à 15 parties d'un additif hydrosoluble ou hydrophile qui est le bicarbonate de sodium pour une partie de la résine acrylique neutre constituant, en suspension aqueuse, l'Eudragit® E 30 D.

12. Nouveaux médicaments, caractérisés en ce qu'ils sont constitués d'au moins un principe actif médicamenteux solide, peu hydrosoluble, et sous forme finement divisée, ledit principe actif étant recouvert d'un enrobage constitué du floculat d'une dispersion aqueuse d'un ou plusieurs agents hydrophobes solides pharmaceutiquement acceptables, lorsque ledit enrobage est réalisé selon le procédé de la revendication 1.

13. Nouveaux médicaments selon la revendication 12, caractérisés en ce que l'agent hydrophobe solide est la résine acrylique neutre constituant, en suspension aqueuse, l'Eudragit® E 30 D.

14. Nouveaux médicaments selon l'une des revendications 12 ou 13, caractérisés en ce que l'enrobage renferme en outre au moins un additif hydrosoluble ou hydrophile.

15. Nouveaux médicaments selon la revendication 14, caractérisés en ce que l'additif hydrosoluble ou hydrophile est choisi dans le groupe constitué par le bicarbonate de sodium et le chlorure de sodium.

16. Nouveaux médicaments selon l'une quelconque des revendications 12 à 15, caractérisés en ce que l'enrobage renferme en outre un agent plastifiant.

17. Nouveaux médicaments selon l'une quelconque des revendications 12 à 16, caractérisés en ce que le principe actif médicamenteux est la propionylérythromycine.

18. Nouveaux médicaments selon les revendications 13 et 17, caractérisés en ce que le poids sec de résine représente de 2 à 15% du poids du principe actif.

19. Compositions pharmaceutiques destinées à la voie orale, caractérisées en ce qu'elles renferment l'un au moins des médicaments tels que définis à l'une quelconque des revendications 12 à 18.

**Revendications** pour l'Etat contractant: AT

1. Procédé d'enrobage d'au moins un principe actif médicamenteux solide peu hydrosoluble et se présentant sous forme finement divisée, caractérisé en ce que l'on mélange sous agitation ledit principe actif et une dispersion aqueuse d'un ou plusieurs agents hydrophobes solides pharmaceutiquement acceptables, fait floculer ledit agent hydrophobe à l'aide d'au moins un moyen physique ou chimique ou une combinaison de ces moyens, sépare le principe actif ainsi enrobé et sèche.

2. Procédé selon la revendication 1, caractérisé en ce que l'agent hydrophobe solide est une résine acrylique.

3. Procédé selon l'une des revendications 1 ou 2, caractérisé en ce que le moyen physique de floculation utilisé est la variation de température.

4. Procédé selon l'une des revendications 1, 2 ou 3, caractérisé en ce que le moyen chimique de floculation utilisé est un électrolyte.

5. Procédé selon la revendication 4, caractérisé en ce que l'électrolyte est le chlorure de sodium.

6. Procédé selon l'une des revendications 1 ou 2, caractérisé en ce que, lors de l'opération de mélange, l'on ajoute en outre un agent mouillant.

7. Procédé selon l'une des revendications 1 ou 2, caractérisé en ce que, lors de l'opération de mélange, l'on ajoute en outre un agent plastifiant.

8. Procédé selon l'une des revendications 1 ou 2, caractérisé en ce que, lors de l'opération de mélange, l'on ajoute en outre un agent antimousse.

9. Procédé selon l'une des revendications 1 ou 2, caractérisé en ce que, lors de l'opération de mélange, l'on incorpore en outre au moins un additif hydrosoluble ou hydrophile.

10. Procédé selon la revendication 2, caractérisé en ce que le mélange renferme environ 1 à 60 parties d'un principe actif qui est la propionylérythromycine pour une partie de résine acrylique.

11. Procédé selon l'une des revendications 9 ou 10, caractérisé en ce que le mélange renferme environ 5 à 15 parties d'un principe actif qui est la propionylérythromycine, et 5 à 15 parties d'un additif hydrosoluble ou hydrophile qui est le bicarbonate de sodium pour une partie de la résine acrylique neutre constituant, en suspension aqueuse, l'Eudragit® E 30 D.

**Patentansprüche** für die Vertragsstaaten BE, CH, DE, GB, IT, LI, NL, SE

1. Verfahren zur Umhüllung von zumindest einem festen wenig wasserlöslichen aktiven Arzneimittelprinzip, das in feinteiliger Form vorliegt, dadurch gekennzeichnet, dass man unter Rühren das aktive Prinzip und eine wässerige Dispersion von einem oder mehreren pharmazeutisch verträglichen festen hydrophoben Mitteln mischt, das hydrophobe Mittel mit zumindest einem physikalischen oder chemischen Mittel oder einer Kombination dieser Mittel ausflocken lässt, das so erhaltene umhüllte aktive Prinzip abtrennt und trocknet.

2. Verfahren gemäss Anspruch 1, dadurch gekennzeichnet, dass das feste hydrophobe Mittel ein Acrylharz ist.

3. Verfahren gemäss Anspruch 1 oder 2, dadurch gekennzeichnet, dass das verwendete physikalische Ausflockungsmittel die Temperaturänderung ist.

4. Verfahren gemäss Anspruch 1, 2 oder 3, dadurch gekennzeichnet, dass das verwendete chemische Ausflockungsmittel ein Elektrolyt ist.

5. Verfahren gemäss Anspruch 4, dadurch gekennzeichnet, dass der Elektrolyt Natriumchlorid ist.

6. Verfahren gemäss Anspruch 1 oder 2, dadurch gekennzeichnet, dass man während des Mischvorgangs ausserdem ein Netzmittel zugibt.

7. Verfahren gemäss Anspruch 1 oder 2 dadurch gekennzeichnet, dass man während des Mischvorgangs ausserdem einen Weichmacher zugibt.

8. Verfahren gemäss Anspruch 1 oder 2, dadurch gekennzeichnet, dass man während des Mischvorgangs ausserdem ein Antischaummittel zugibt.

9. Verfahren gemäss Anspruch 1 oder 2, dadurch gekennzeichnet, dass man während des Mischvorgangs ausserdem zumindest ein wasserlösliches oder hydrophiles Additiv einbringt.

10. Verfahren gemäss Anspruch 2, dadurch gekennzeichnet, dass das Gemisch etwa 1-60 Teile eines aktiven Prinzips, bei dem es sich um das Propionylerythromycin handelt, je 1 Teil des Acrylharzes umfasst.

11. Verfahren gemäss Anspruch 9 oder 10, dadurch gekennzeichnet, dass das Gemisch etwa 5-15 Teile eines aktiven Prinzips, bei dem es sich um das Propionylerythromycin handelt, und 5-15 Teile eines wasserlöslichen oder hydrophilen Additivs bei dem es sich um das Natriumbicarbonat handelt, je 1 Teil des neutralen Acrylharzes, das in wässeriger Suspension das Eudragit® E 30 D darstellt, umfasst.

12. Neue Arzneimittel, dadurch gekennzeichnet, dass sie zumindest aus einem festen, wenig wasserlöslichen aktiven Arzneimittelprinzip in feinteiliger Form bestehen, wobei das aktive Prinzip mit einer Umhüllung überzogen ist, die aus dem Flockulat einer wässerigen Dispersion von einem oder mehreren pharmazeutisch verträglichen festen hydrophoben Mitteln besteht, wobei die Umhüllung gemäss dem Verfahren nach Anspruch 1 erfolgt ist

13. Neue Arzneimittel gemäss Anspruch 12, dadurch gekennzeichnet, dass das feste hydrophobe Mittel das neutrale Acrylharz ist, das in wässeriger Suspension das Eudragit® E 30 D darstellt.

14. Neue Arzneimittel gemäss Anspruch 12 oder 13, dadurch gekennzeichnet, dass die Umhüllung ausserdem zumindest ein wasserlösliches oder hydrophiles Additiv umfasst.

15. Neue Arzneimittel gemäss Anspruch 14, dadurch gekennzeichnet, dass das wasserlösliche oder hydrophile Additiv unter Natriumbicarbonat oder Natriumchlorid ausgewählt ist.

16. Neue Arzneimittel gemäss einem der Ansprüche 12-15, dadurch gekennzeichnet, dass die Umhüllung ausserdem einen Weichmacher umfasst.

17. Neue Arzneimittel gemäss einem der Ansprüche 12-16, dadurch gekennzeichnet, dass das aktive Arzneimittelprinzip das Propionylerythromycin ist.

18. Neue Arzneimittel gemäss Anspruch 13 und 17, dadurch gekennzeichnet, dass das Trockengewicht des Harzes 2-15% des Gewichts des aktiven Prinzips ausmacht.

19. Pharmazeutische Zusammensetzungen für die orale Verabreichung, dadurch gekennzeichnet, dass sie zumindest eines der Arzneimittel gemäss einem der Ansprüche 12-18 umfassen.

**Patentansprüche** für den Vertragsstaat AT

1. Verfahren zur Umhüllung von zumindest einem wenig wasserlöslichen festen aktiven Arzneimittelprinzip in feinteiliger Form, dadurch gekennzeichnet, dass man unter Rühren das aktive Prinzip und eine wässerige Dispersion von einem oder mehreren pharmazeutisch verträglichen festen hydrophoben Mitteln mischt, das hydrophobe Mittel mit Hilfe zumindest eines physikalischen oder chemischen Mittels oder einer Kombination dieser Mittel ausflocken lässt, das so umhüllte aktive Prinzip abtrennt und trocknet.

2. Verfahren gemäss Anspruch 1, dadurch gekennzeichnet, dass das feste hydrophobe Mittel ein Acrylharz ist.

3. Verfahren gemäss Anspruch 1 oder 2, dadurch gekennzeichnet, dass verwendete physikalische Ausflockungsmittel die Temperaturänderung ist.

4. Verfahren gemäss Anspruch 1, 2 oder 3, dadurch gekennzeichnet, dass das verwendete chemische Ausflockungsmittel ein Elektrolyt ist.

5. Verfahren gemäss Anspruch 4, dadurch gekennzeichnet, dass der Elektrolyt Natriumchlorid ist.

6. Verfahren gemäss Anspruch 1 oder 2, dadurch gekennzeichnet, dass man während des Mischvorgangs ausserdem ein Netzmittel zugibt.

7. Verfahren gemäss Anspruch 1 oder 2, dadurch gekennzeichnet, dass man während des Mischvorgangs ausserdem einen Weichmacher zugibt.

8. Verfahren gemäss Anspruch 1 oder 2, dadurch gekennzeichnet, dass man während des Mischvorgangs ausserdem ein Antischaummittel zugibt.

9. Verfahren gemäss Anspruch 1 oder 2, dadurch gekennzeichnet, dass man während des Mischvorgangs ausserdem zumindest ein wasserlösliches oder hydrophiles Additiv einbringt.

10. Verfahren gemäss Anspruch 2, dadurch gekennzeichnet, dass das Gemisch etwa 1-60 Teile eines aktiven Prinzips, bei dem es sich um das Propionylerythromycin handelt, je 1 Teil des Acrylharzes umfasst.

11. Verfahren gemäss Anspruch 9 oder 10, dadurch gekennzeichnet, dass das Gemisch etwa 5-15 Teile eines aktiven Prinzips, bei dem es sich um das Propionylerythromycin handelt, und 5-15 Teile eines wasserlöslichen oder hydrophilen Additivs, bei dem es sich um das Natriumbicarbonat handelt, je 1 Teil des neutralen Acrylharzes, das in wässeriger Suspension das Eudragit® E 30 D darstellt, umfasst.

**Claims** for the contracting States: BE, CH, DE, GB, IT, LI, NL, SE

1. Process for coating at least one solid medicament active principle, slightly soluble in water and presented in a finely divided form, characterized in that the said active principle and an aqueous dispersion of one or more pharmaceutically acceptable solid hydrophobic agents are mixed together under agitation, the said hydrophobic agent is made to flocculate by a physical or chemical means or a combination of these means, the active principle so coated is separated and dried.

2. Process according to claim 1, characterized in that the solid hydrophobic agent is an acrylic resin.

3. Process according to claim 1 or 2, characterized in that the physical means of flocculation utilized is a variation of temperature.

4. Process according to claim 1, 2 or 3, characterized in that the chemical means of flocculation utilized is an electrolyte.

5. Process according to claim 4, characterized in that the electrolyte is sodium chloride.

6. Process according to claim 1 or 2, characterized in that during the mixing operation a wetting agent is also added.

7. Process according to claim 1 or 2, characterized in that during the mixing operation a plastifying agent is also added.

8. Process according to claim 1 or 2, characterized in that during the mixing operation an antifoaming agent is also added.

9. Process according to claim 1 or 2, characterized in that during the mixing operation at least one hydrosoluble or hydrophilic additive is also incorporated.

10. Process according to claim 2, characterized in that the mixture contains about 1 to 60 parts of an active principle which is propionylerythromycine for one part of acrylic resin.

11. Process according to claim 9 or 10, characterized in that the mixture contains about 5 to 15 parts of an active principle which is propionylerythromycine, and 5 to 15 parts of a hydrosoluble or hydrophilic additive which is sodium bicarbonate for one part of the neutral acrylic resin, constituting, in aqueous suspension, Eudragit® E 30 D.

12. New medicaments characterized in that they are constituted by at least one solid medicament active principle, slightly soluble in water and in a finely divided form, the said active principle being covered with a coating constituted by the flocculate of an aqueous dispersion of one or more pharmaceutically acceptable solid hydrophobic agents, when the said coating is carried out according to the process of claim 1.

13. New medicaments according to claim 12, characterized in that the solid hydrophobic agent is the neutral acrylic resin constituting, in aqueous suspension, Eudragit® E 30 D.

14. New medicaments according to claim 12 or 13, characterized in that the coating also contains at least one hydrosoluble or hydrophilic additive.

15. New medicaments according to claim 14, characterized in that the hydrosoluble or hydrophilic additive is chosen from the group constituted by sodium bicarbonate and sodium chloride.

16. New medicaments according to any one of claims 12 to 15, characterized in that the coating contains also a plastifying agent.

17. New medicaments according to any one of claims 12 to 16, characterized in that the medicament active principle is propionylerythromycine.

18. New medicaments according to claims 13 and 17, characterized in that the dry weight of the resin represents from 2 to 15% of the weight of the active principle.

19. Pharmaceutical compositions intended for oral route, characterized in that they contain one at least of the medicaments as defined in any one of claims 12 to 18.


**Claims** for the contracting State: AT

1. Process for coating at least one solid medicament active principle, slightly soluble in water and presented in a finely divided form, characterized in that the said active principle and an aqueous dispersion of one or more pharmaceutically acceptable solid hydrophobic agents are mixed together under agitation, the said hydrophobic agent is made to flocculate by a physical or chemical means or a combination of these means, the active principle so coated is separated and dried.

2. Process according to claim 1, characterized in that the solid hydrophobic agent is an acrylic resin.

3. Process according to claim 1 or 2, characterized in that the physical means of flocculation utilized is a variation of temperature.

4. Process according to claim 1, 2 or 3, characterized in that the chemical means of flocculation utilized is an electrolyte.

5. Process according to claim 4, characterized in that the electrolyte is sodium chloride.

6. Process according to claim 1 or 2, characterized in that during the mixing operation a wetting agent is also added.

7. Process according to claim 1 or 2, characterized in that during the mixing operation a plastifying agent is also added.

8. Process according to claim 1 or 2, characterized in that during the mixing operation an antifoaming agent is also added.

9. Process according to claim 1 or 2, characterized in that during the mixing operation at least one hydrosoluble or hydrophilic additive is also incorporated.

10. Process according to claim 2, characterized in that the mixture contains about 1 to 60 parts of an active principle which is propionylerythromycine for one part of acrylic resin.

11. Process according to claim 9 or 10, characterized in that the mixture contains about 5 to 15 parts of an active principle which is propionylerythromycine, and 5 to 15 parts of a hydrosoluble or hydrophilic additive which is sodium bicarbonate for one part of the neutral acrylic resin, constituting, in aqueous suspension, Eudragit® E 30 D.